# EUROPEAN PATENT APPLICATION

(11) **EP 4 628 052 A1**
(43) Date of publication of application: **08.10.2025**
(21) Application number: 24168116.2
(22) Date of filing: 02.04.2024
(51) Int. Cl.: A61F 2/14, A61F 9/007

(54) **INTRAOCULAR LENS CAPSULE KIT FEATURING CAPSULE DRAINAGE**

(71) Applicant: Vatavuk, Zoran, 10000 Zagreb (HR); Maric, Goran, 10000 Zagreb (HR)
(72) Inventor: Vatavuk, Zoran, 10000 Zagreb (HR); Maric, Goran, 10000 Zagreb (HR)
(74) Representative: Bihar, Zeljko

(57) **Abstract**

An intraocular lens capsule kit featuring posterior chamber drainage is disclosed. In preferred embodiment, it comprises a lens capsule (10), formed as a toroidal body with a C-shaped cross-section, at least three capsule arms (20), where at least one capsule arm (20) has a haptic end for sutureless scleral fixation, formed on the one end of the arm duct with the insertion tip formed on the opposite end of the duct to be inserted into the capsule. Optionally, the kit has one or more filtration blebs (30), capable of being connected with any haptic end of the capsule arm (20), and, if necessary, an artificial iris that can be detachably mounted on the said capsule. The arm duct has capillary made through the tip towards the haptic end and enables the effective drainage of the posterior chamber where the capsule (10) is implanted, with or without the filtration bleb (30).

## Description

### Technical Field

The present disclosure relates to an intraocular lens capsule kit, for accommodation of the artificial lens or implantable telescope lens, with the ability to provide drainage from the posterior chamber to the episcleral space. The technical field is related to artificial substitutes or replacements for body parts and appliances to connect them with the body, i.e., where the body or body parts relate to the patient's eye and the parts thereof.

### Technical problem

There is a growing need in ophthalmology to handle complex anterior segments conditions adeptly. Among others, this includes a secondary intraocular lens (IOL) implantations regarding lens capsule and zonules defects following trauma, congenital malformations or surgery injuries, as well as iris defects following trauma or congenital malformations and also increased eye pressure in conditions like primary or secondary glaucoma and eye hypertension.

Some or even all of the above problems occur simultaneously with some diseases and after injuries to the eyeball. For example, the combination of lens capsule and zonule damage with increased intraocular pressure is particularly evident in PEX glaucoma; or aphakia, iris defect, and secondary glaucoma are present following some (penetrant) eye injuries altogether. There is no single technique and device to solve all of these pathological entities at the same time.

There are several surgical techniques to deal with these problems individually and separately, but each of them has its complications and disadvantages such as: inappropriate refraction outcome because of intraocular lens tilt or other malposition (scleral IOL fixation techniques), secondary glaucoma, uveitis, hyphaema, pigment dispersion syndrome, corneal endothelium damage, anterior chamber IOL's, iris-claw IOL's, glaucoma devices with tubes in anterior chamber, artificial iris suturing to the sclera or remaining natural iris tissue, etc.

The main technical problem solved with this invention is simultaneous sutureless optimal positioning of intraocular lens, or telescope, in the posterior chamber of the eye as well as therapeutic decrease of intraocular pressure, through an alternative drainage route from the posterior chamber directly to the episcleral space, bypassing anterior chamber at all, and, if needed, atraumatic positioning of the artificial iris in its natural position.

Above all, in this way, there is a pleasant situation for possible elegant replacement of the IOL implanted nowadays with some potentially better quality IOL in the future. Using this invention, all the abovementioned problems could be solved simultaneously and with less postoperative complications compared to previous techniques.

It is understood then that different parts of this unified solution can be combined making this solution even more modular, e.g., intraocular lens implantation with intraocular pressure decrease or artificial iris implantation with intraocular lens implantation.

There is a possibility of subsequent addition of parts of this invention using one part as a platform for implanting another one, during additional surgery, optionally and if needed.

### Prior Art

The prior art documents are focused on solving only one problem, i.e., the intraocular lens capsule fixation, or the drainage of the aqueous humor *per se,* only from the anterior chamber.

European patent application EP3870109 for the invention INTRAOCULAR ARTIFICIAL LENS CAPSULE, filed in the name of The Board of Trustees of the Leland Stanford Junior University, US. It discloses an implantable artificial capsule, with at least three haptic arms configured to sit sub-conjunctivally upon implantation in the eye. Also, each of the arms comprises a trans-scleral anchor for sutureless trans-scleral fixation upon implantation. The cited document is silent regarding drainage, and it is focused only on the sutureless fixation.

European patent EP3010445 for the invention SCLERAL FIXATION BAG, filed in the name of VisonCare, Inc, US. It discloses a scleral fixation bag with haptics that extend therefrom, to be secured by sclerotomy. The bag is designed to receive an intraocular lens or implantable telescope but remains silent regarding the drainage.

US patent US 5,628,795 for the invention SPARE PARTS FOR USE IN OPHTHALMIC SURGICAL PROCEDURES, filed in the name of David W. Langerman, US. It reveals ophthalmic 'spare parts' made of biocompatible material that may be implanted in the ciliary sulcus, or the residual natural capsular bag of a patient's eye. The text reveals the capsule with a haptic part formed as resilient loops, arms, or the like.

European patent EP1333879 for the invention C-SHAPED CROSS SECTION TUBULAR OPHTHALMIC IMPLANT FOR REDUCTION OF INTRAOCULAR PRESSURE IN GLAUCOMATOUS EYES, filed in the name of Michael J. Wilcox, US. In one of its invention aspects, it provides a method for reducing intraocular pressure by creating a cylindrical bleb for producing improved accessory filtration by implanting a cylindrical tube having a proximal end and distal end, the distal portion of the cylindrical tube side wall being removed, into an eye to serve as a nidus for a conduit of aqueous humor to bypass angle structures. However, this document is silent regarding any artificial lens capsule implantation, and the eye pressure is reduced by acting on the anterior eye chamber. The document is silent regarding the posterior chamber drainage.

Similarly, US patent US 7,431,709 for the invention GLAUCOMA IMPLANT DEVICE, filed in the name of Innfocus LLC, US. It discloses an implant device for treating glaucoma that includes an elongated duct structure, formed from a material comprising polyisobutylene and a glassy segment. The material of the elongate duct structure preferably has a general block structure with a central elastomeric polyolefinic block and thermoplastic end blocks (e.g., a triblock polymer backbone comprising polystyrene-polyisobutylene-polystyrene). The elongated duct structure provides a fluid passageway for diverting aqueous humor from the anterior chamber of the eye. Again, this product is silent regarding any other implants, such as an intraocular lens capsule.

Kerr, N.M., Ahmed, I.I.K., Pinchuk, L., Sadruddin, O., Palmberg, P.F. (2021). PRESERFLO MicroShunt. In: Sng, C.C.A., Barton, K. (eds) Minimally Invasive Glaucoma Surgery. Springer, Singapore. https://doi.org/10.1007/978-981-15-5632-6_7. This article describes the PRESERFLO^{®} MicroShunt, Santen Pharmaceutical Co. Ltd., Osaka, Japan, formerly called the InnFocus MicroShunt^{®}, a trans-scleral device that shunts aqueous humor from the anterior chamber to a filtering bleb under the conjunctiva and Tenon's capsule.

Even, very recent works are concentrated only on the drainage of the anterior chamber, being silent about the drainage of the posterior eye's chamber. US patent US 10,307,292, for the invention Device FOR ADJUSTING THE INTRAOCULAR PRESSURE, filed in the name of Mor Research Application Ltd., IL, discloses methods of draining aqueous humor from the anterior chamber to the intra-orbital space.

According to the best knowledge, it seems that the unique solution for accommodating the intraocular lens capsule with the posterior chamber drainage via one of its haptic arms is not revealed in the prior art. In addition, the solution where the drainage duct, formed in the haptic arms, ends in the filtration bleb that is situated at the haptic end, has not been reported earlier. Furthermore, the disclosed kit enables the artificial iris to be easily implemented over the said lens capsule as a clear advantage over the prior art.

### Summary

An intraocular lens capsule kit featuring capsule drainage is disclosed. Minimally, it comprises a lens capsule and at least three capsule arms.

The lens capsule is designed for accommodating the artificial lens or implantable telescope lens. It is formed as a toroidal body with a C-shaped cross-section, with an inner concave surface and an outer convex surface. The capsule is equipped with at least three bores connecting the said surfaces. The said capsule has, optionally, on its outer surface at least two pegs positioned to intersect the plane laying parallel over the entire top of the C-shaped cross-section.

Among the capsule arms, at least one capsule arm has a haptic end for suture-less scleral fixation formed on the one end of the arm duct, with the insertion tip formed on the opposite end of the duct. The connecting means, which is formed close to the tip, is used to connect the said capsule arm and the capsule by inserting the tip into the selected bore.

The said kit has, optionally, one or more filtration blebs, or, bleb elements - an outer surface and a base. The bleb's base is capable of being connected via its receiving slit with the haptic end of the capsule arm. Optionally, the kit has an artificial iris with slits distributed around the iris to correspond with the capsule's pegs geometry and enable iris fixation to the said capsule.

The kit specific is that the arm duct has a capillary made through the tip towards the haptic end, enabling effective drainage from the posterior chamber and anterior vitreous space, situated in the posterior chamber, to the episcleral space directly or through a scleral tunnel in pocket form.

In the preferred embodiment, the connecting means is formed as a recess on the duct, situated close to the insertion tip. The recess has a diameter that corresponds to the bore diameter, while the duct has a slightly greater diameter than the bore diameter, ensuring the locking of the said recess within the capsule. In addition, the haptic end is elliptic or round and inclined for 15°-45° towards the duct, so that the haptic end follows the sclera's curvature and sticks with it on the surface of the sclera or in the scleral pocket.

In yet another embodiment, the capsule's bores are distributed equidistantly over the capsule's toroidal body.

In the most preferred embodiment, the capsule has three bores and three capsule arms with the haptic end. In yet another preferred embodiment, the kit further comprises a capsule with two pegs and an artificial iris with three slits that match the position of the said pegs.

In yet another embodiment, the kit further comprises a filtration bleb formed from the elements; an outer surface equipped with the spacers, and a base detachable coupled to the said outer surface. The bleb's base and the outer surface couplings are achieved via insets formed on the bleb's surface edge, which engage the slits formed on the bleb's base. In one variant, the bleb is further equipped with one or more holes made on the bleb's base, for an auxiliary fixation of the said base to the sclera by surgical sutures. The bleb's interior, formed between the outer surface and the base can be used as a drug reservoir, or depo for slow release of the selected medicament.

In yet another embodiment, the kit elements are coated with suitable agents, selected from anti-inflammatory, anti-glaucomatous, anti-VEGF, or antifibrotic agents or their combinations.

The intraocular lens capsule kit featuring drainage is used for providing the drainage support for the artificial lens situated within the capsule fixed to the sclera via the haptic ends of the capsule arms, and, optionally, equipped with at least one filtration bleb mounted on one of the haptic ends of the capsule arm and with the artificial iris, if necessary.

### A brief description of the figures

Figure 1 shows a toroidal body with a C-shaped cross-section, i.e., a capsule - with evenly distributed bores. Figure 2 shows the said C-shaped cross-section across the capsule.
Figure 3 depicts a cross-section of the haptic end connection with the capsule.
Figure 4 depicts one kit's preferred embodiment, three arms with haptic ends, and the capsule ready to receive it. Figure 5 shows the completed functional device - an intraocular lens capsule with mounted arms with the haptic ends.
Figure 6 depicts the variant of the capsule shown in Figure 1, with three pegs for receiving the artificial iris. Figures 7 and 8 are variants of the situation represented in Figures 4 and 5, with pegs added to the capsule.
Figure 9 shows the process of artificial iris fixation to the completed functional device - an intraocular lens capsule with mounted arms with haptic ends.
Figure 10 shows the cross-section of the trinary system, i.e., the bleb - the arm with the haptic end - the intraocular capsule. Figure 11 shows the same trinary system and its constituting parts.
Figure 12 depicts the bleb-arm-capsule connection. Figure 13 represents the preferred embodiment with the bleb, implanted in an eye. Figure 14 represents the preferred embodiment with the bleb, implanted in an eye and with the intraocular lens positioned within the capsule.
Figure 15 depicts the haptic end situated within the episcleral space, and Figure 16 depicts the haptic end situated in the scleral pocket, under the scleral flap.

### Detailed description of the disclosure

An intraocular lens capsule kit featuring capsule drainage is disclosed. Minimally it comprises a lens capsule and at least three capsule arms, with some additional kit elements that can be easily combined for maximum performance.

### The capsule

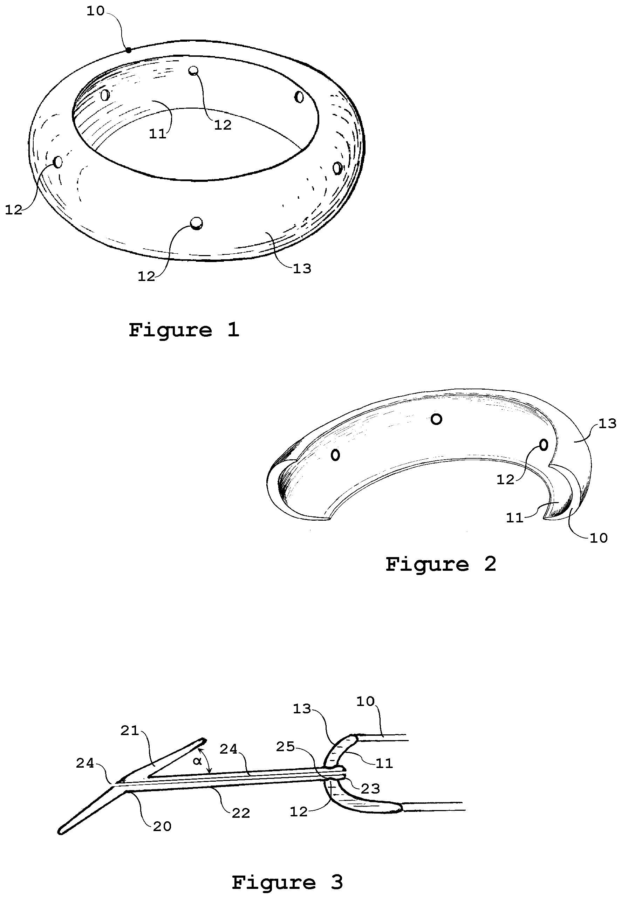

The capsule is well known from the prior art and is used for accommodating the artificial lens or implantable telescope lens. They are an artificial replacement for the lens of the patient's eye and do not constitute the essential kit elements disclosed herein. The capsule (10) is depicted in Figure 1 in a most general way. It is formed as a toroidal body with a C-shaped cross-section as depicted in Figure 2, with an inner concave surface (11) and outer convex surface (13). The capsule (10) is equipped with at least three bores (12) which connect the said surfaces (11, 13). The bores (12) may be distributed arbitrarily, however, the preferred embodiment has evenly distributed bores (12) to equalize the tensions exerted on the capsule body issued by the capsule arms (20) connected thereto. In practice, the preferred embodiment consists of three bores (12).

Even though it is possible to imagine the capsule (10) with two bores (12), formed on the opposite sides of the C-shaped body, such construction fails in real implementation. First, it is hard to fix the capsule (10) with two capsule arms to the desired position, without subsequent lateral movements. Namely, capsule arms produce tensions on the opposite capsule's sides and even small lateral forces will significantly move the capsule (10) within the equilibrium defined only by two fixation points. Secondly, such tensions may deform the capsule (10) shape, in case that the capsule material is not rigid enough, which may prevent easy insertion of the artificial lens or implantable telescope lens. That problem is well recognized in the cited prior art documents, and all documents mentioned at least three fixation points of the capsule, preferably evenly distributed rendering the uniform load on the capsule (10).

Regarding the capsule size, in more than 90% one size will fit. In extreme dioptres, above +8 and below -8 the different capsule (10) sizes can be used to better accommodate the eye's size. Optimal size could be adjusted according to the specific biometric eye parameters, white-to-white (WTW), axial length of the eye (AL) etc.

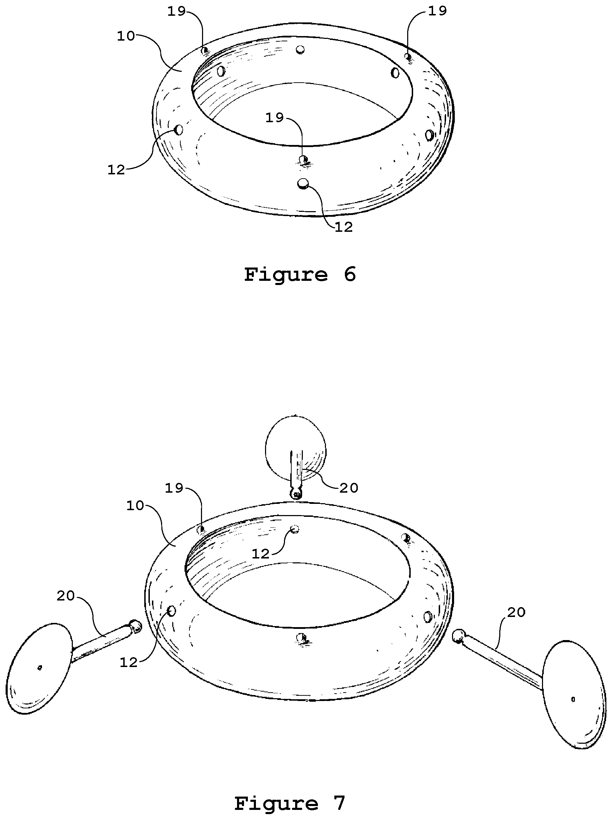

In one variant, the capsule (10) has on its outer surface (13) at least two pegs (19) as depicted in Figure 6. The pegs (19) are positioned to intersect the plane laying parallel over the entire top of the C-shaped cross-section. They are designed, if necessary, to serve as the anchors for the artificial iris (90), which we will describe later.

### The capsule arms with connecting means

The role of the capsule arms (20) is to be connected with the capsule (10), once the capsule is positioned within the space left by the removed or missing eye lens and the corresponding natural capsule. The artificial capsule arms (20) have a role similar to the zonular attachments, i.e., to support the artificial capsule (10) in the desired position.

According to the invention, the kit comprises at least three capsule arms (20), where at least one capsule arm (20) is designed to enable drainage, and where all arms (20) are removably connected with the capsule (10). Figure 3 depicts the cross-section of such a capsule arm (20). The capsule arm (20) has a haptic end (21) for suture-less scleral fixation formed on the one end of the arm duct (22). The haptic end (21) is elliptic or circular and inclined for an angle α (15°-45°) towards the duct (22), so that the haptic end (21) follows the sclera's curvature, as depicted in Figure 3. The insertion tip (23) is formed on the opposite end of the duct (22) and designed to be removably attached to the capsule (10), using friction or other methods to secure the said tip (23) within the capsule's bore (12) . In the preferred embodiment, the connecting means (25), is formed close to the tip (23) to connect the said capsule arm (20) and the capsule (10). The connecting means (25) is preferably formed as a recess on the duct (22), situated close to the insertion tip (23). The recess (25) has a diameter that corresponds to the bore (12) diameter, while the duct (22) has a slightly greater diameter than the bore (12) diameter ensuring the locking of the said recess (25) within the capsule (10). The preferred duct's cross-section is circular, but other cross-sections can be used equally well. It is possible to imagine other connecting means (25), for instance, a pure frictional connecting means (25) without recess, but such modification seems to be obvious to the person skilled in the art.

The main feature of the described capsule arms with connecting means is that the duct (20) has capillary (24) made through the tip (23) towards the haptic end (21). The capillary (24) enables the effective drainage of the aqueous humor, from the lens capsule (10) situated in the posterior chamber to the episcleral space. The capsule arms (20) can have different capillary (24) diameters if needed for some specific (therapeutic) purposes.

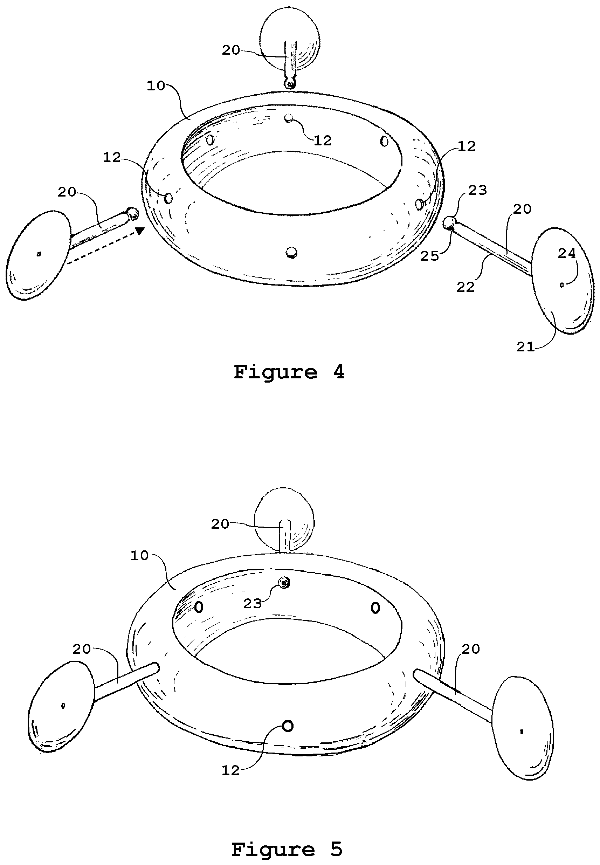

In practice, it is desirable to use all capsule arms (20) with drainage ability, preferably with the same capillary diameters. The practical way of assembling such a kit is depicted in Figures 4 and 5.

### The artificial iris

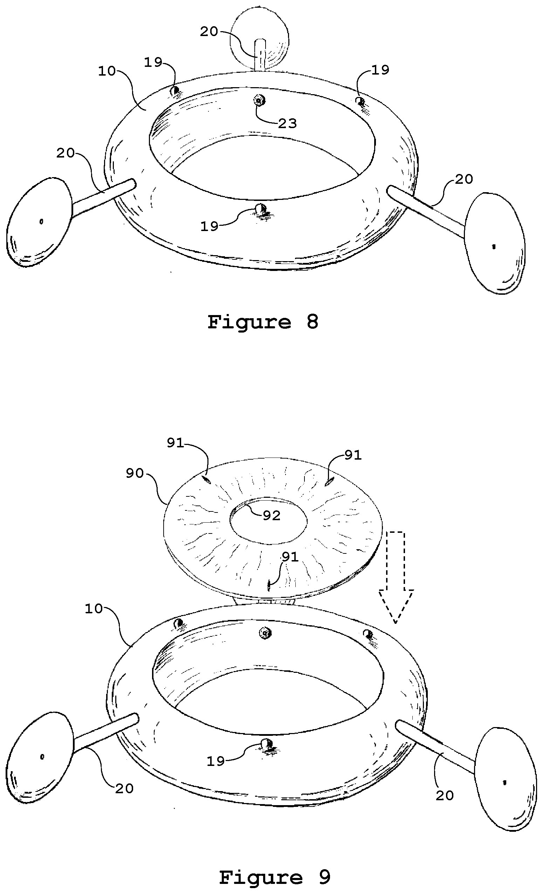

The artificial iris (90) is not a mandatory kit element. Figure 9 depicts the iris (90), with the central aperture (92), and iris slits (91) designed to be attached to the pegs (19) formed on the capsule (10). Figure 7-9 shows the assembly steps, where in the last step the artificial iris (90) is connected, via its slits (91), with the capsule's pegs (19).

The person skilled in the art will certainly recognize other suitable mechanisms for attaching the artificial iris (90) to the capsule (10). For instance, it can be an opposite variant of those disclosed above, i.e., where the iris (90) is equipped with pegs that can be accommodated to the bores formed on the capsule (10) for receiving such pegs.

### The filtration bleb

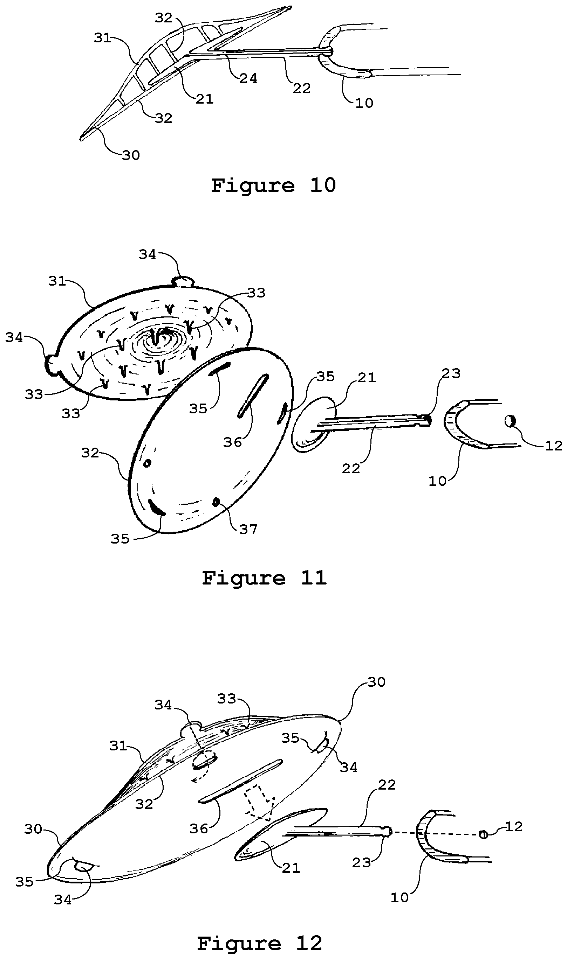

The filtration bleb (30) is not a mandatory kit element, and the number of filtration blebs (30) within the kit varies from zero to the number of capsule arms used. The bleb's role is to serve as a filtration means to receive an excess of the aqueous humor if the standard drainage via the capsule arms (20) is not sufficient due to the pressures exerted on their haptic end. In that case, the bleb has the role of the expansion chamber, i.e., to accumulate the sudden aqueous humor excess. Figure 10 shows the cross-section of the system bleb-arm-capsule, and Figure 11 depicts the bleb (30) construction.

The bleb (30) is formed from an outer surface (31) equipped with spacers (33) and a base (32) detachable coupled to the said outer surface (31), as depicted in Figure 11. The base (32) and the surface (31) are coupled via insets (34) formed on the surface edge, which engage the slits (35) formed on the base (32). The spacers (33) define the bleb's interior volume. The bleb's base (32) is equipped with the receiving slit (36), designed to accommodate the haptic end (21) of the capsule's arm (20), as depicted in Figure 10. The bleb's interior formed between the outer surface (31) and the base (32) can be used as a drug reservoir, or depo for slow release of the medicament, where the drug or medicament is selected according to the patient's need.

Optionally, the bleb (30) is further equipped with one or more holes (37), made on the base (32), for an auxiliary fixation of the said base to the sclera by surgical sutures to prevent the bleb movements.

According to the disclosure, the bleb is preferably formed *in situ* during the surgical procedure, so, in the preferred embodiment the kit comprises the bleb elements outer surface (31) and the base (22). One or more blebs (30) are, preferably, situated in the episcleral space (150) as well.

### Material selection for the kit

The person skilled in the art will certainly select the suitable material for the kit formation, known in the prior art.

All kit elements may be made from the same or different materials, and each may be made of one or more materials. The materials used are biocompatible and, if necessary, optically transparent. The selected materials can be hydrophilic or hydrophobic. The material may be rigid or flexible, hard or soft such as without limitation, methacrylates (e.g., polymethyl methacrylate), olefins (e.g., polypropylene), and silicones.

Used materials can be all, or in part, coated with the suitable agents, selected from anti-inflammatory, anti-glaucomatous, anti-VEGF, or antifibrotic agents or their combinations. The antifibrotic agent should be selected to be, preferably, mitomycin-c or 5-fluorouracil, to prevent scar occurrence.

### Surgical procedures

The surgical procedures are briefly described below.

After standard preoperative preparation, an anterior vitrectomy or even a meticulous pars plana vitrectomy is performed. Any remnants of the natural lens are removed. The conjunctiva and the Tenon's capsule are separated to an extent sufficient to allow access to the desired scleral positions provided for each of the haptics - a capsule arm with the haptic end (20), e.g., pars plana positions at 12 o'clock, 4 o'clock and 8 o'clock. If necessary, a peritomy can be performed along the entire circumference of the limbus, 360°.

The main corneoscleral incision is then performed as usual during phacoemulsification cataract surgery with the protection of the corneal endothelium using an ophthalmic viscosurgic device (OVD).

The capsule (10) is injected into the eye through the corneoscleral incision using a custom injector or an existing one that is usually used for intraocular lens implantation in the eye. It is positioned in the anterior eye chamber or partially in the posterior eye chamber through the pupil. In the case of complete aniridia or a significant partial iris defect, it is positioned in the vitreous space. Using forceps inserted through a corneoscleral incision or a previously formed pars plana scleral entry - with or without a pars plana trocar, the capsule (10) is brought into the desired position and held in place. On the other hand, an opening is made on the sclera on the pars plana for the insertion of the capsule arm (20) using, e.g., trocar guide as is standardly used for pars plana vitrectomy - and this arm (20) is inserted transscleral using external forceps. The capsule arm (20) is connected to the capsule (10) by inserting the tip (23) into the opening bore (12). The procedure is repeated for the connection with the second and third, and each subsequent capsule arm (20).

Target dosing of postoperative intraocular pressure decrease can be achieved by choosing a different number of capsule arms (20), due to the different number and effect of capillaries (24) where each capillary *per se* contributes to pressure decrease.

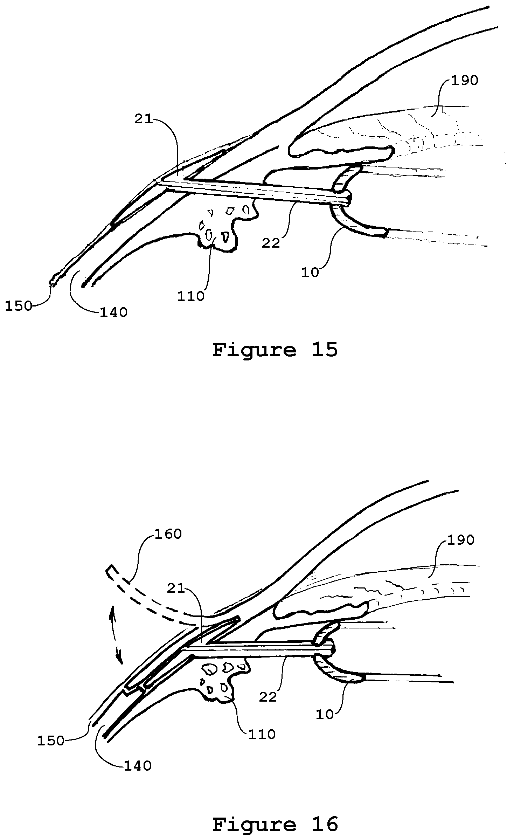

Arms with haptics end (20), implanted in this way may be sufficient for adequate postoperative lowering of intraocular pressure. The haptic end can be placed in the episcleral space and lie directly on the sclera (140), or it can also be placed in a deeper layer of the sclera, or placed in a scleral pocket below the scleral flap (160) that has open communication with the episcleral space (150) . The scleral flap (160) formation is well described in the reference:
Stem, M. S., Todorich, B., Woodward, M. A., Hsu, J., & Wolfe, J. D. (2017). Scleral-Fixated Intraocular Lenses. Journal of VitreoRetinal Diseases, 1(2), 144-152. doi:10.1177/2474126417690650
and these two techniques, where the haptic end (21) is placed in the episcleral space (150) or below the scleral flap (160) are depicted in Figures 15 and 16.

If the preoperative pressure is so high that a more significant, additional postoperative lowering of the intraocular pressure is required, a filtration bleb (30) is attached to one of the haptic ends (21). It is preferably positioned at 12 o'clock for later postoperative covering with the upper eyelid. The underlying and near surrounding scleral tissue at that site can be treated with short-term topical mitomycin-c to reduce the postoperative probability of fibrosis of the subconjunctival space around the bleb.

The lower sheet of the bleb, i.e., the base (32) is attached first by inserting it around the haptic end (21) through the receiving slit (36). In the next step, the outer surface (31) is attached to the base (32) by inserting the inset (34) into the slits (35). The bleb's base (32) can be additionally attached to the sclera by stitching through the provided openings, i.e., stitching holes (37).

Intraocular lens (IOL) is implanted in the capsule through the corneoscleral incision using an injector as in standard cataract surgery procedure.

If artificial iris (90) implantation is needed, it is implanted into the eye folded through the previously mentioned main corneoscleral incision using an injector, then it is unfolded after releasing in the anterior eye chamber and attached to the pegs (19) for the iris slits (91).

The operation is completed by washing out the viscosurgic device (OVD) from the anterior chamber, instilling an antibiotic, e.g., cefuroxime, and sealing the corneoscleral incision by hydrating it with a blunt needle. The peritomy is sewn with resorbable sutures. Dexamethasone is optionally injected subconjunctivally. Antibiotic ointment is applied, e.g., tobramycin and a sterile bandage is placed on the closed eye until the next day.

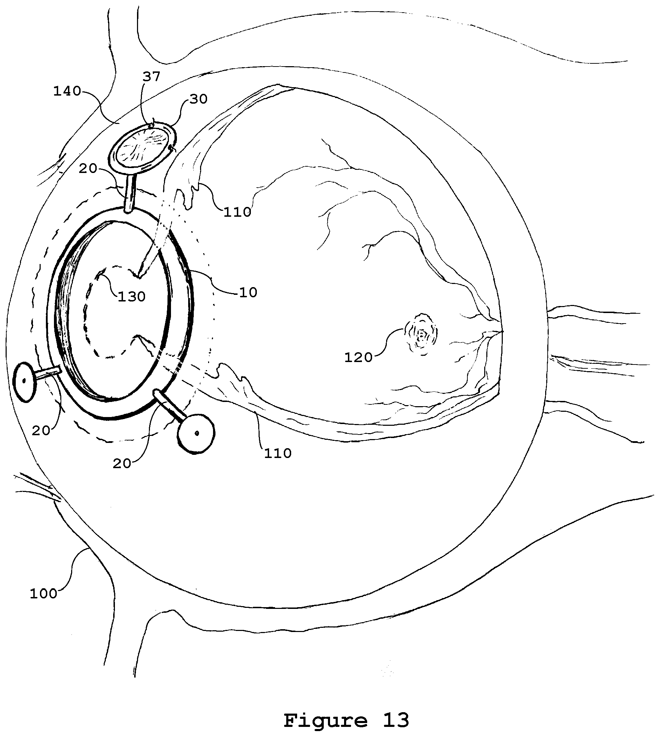

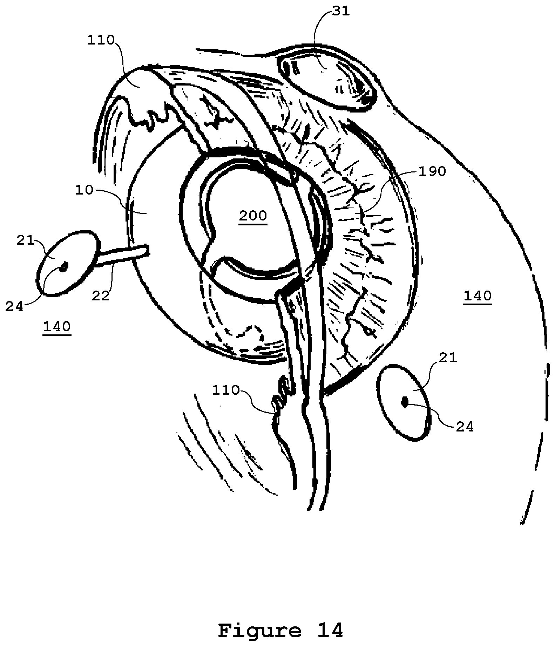

Figure 13 represents the preferred embodiment with the bleb, implanted in an eye according to the above-described procedure. Figure 14 represents the preferred embodiment with the bleb, implanted in an eye and with the intraocular lens (200) positioned within the capsule (10). Figures depict only the essential parts of the eye (100) anatomy, a ciliary muscle (110), an optic disk (120), a pupil (130), a sclera (140), and an iris (190).

### Industrial Applicability

The present disclosure relates to an intraocular lens capsule kit, for the accommodation of the artificial lens or implantable telescope lens, with the ability to provide drainage from the posterior chamber to the episcleral space, and implanting artificial iris. The industrial applicability of the disclosure is obvious.

### References

- 10: Capsule
- 11: Inner surface
- 12: Bore
- 13: Outer surface
- 19: Peg
- 20: Capsula arm with haptic end
- 21: Haptic end
- 22: Duct
- 23: Tip
- 24: Capillary
- 25: Recess
- 30: Filtration bleb
- 31: Outer surface
- 32: Base
- 33: Spacer
- 34: Inset
- 35: Slit
- 36: Receiving slit
- 37: Stitching hole
- 90: Artificial Iris
- 91: Iris slit
- 92: Aperture
- 100: Eye
- 110: Ciliary muscle
- 120: Optic disk
- 130: Pupil
- 140: Sclera
- 150: Episcleral (subconjunctival) space
- 160: Scleral flap
- 190: Iris
- 200: Artificial lens

## Claims

1. An intraocular lens capsule kit featuring capsule drainage, which comprises:
- a lens capsule (10) for accommodating the artificial lens or implantable telescope lens, which is formed as a toroidal body with C-shaped cross-section, with an inner concave surface (11) and outer convex surface (13), where the capsule (10) is equipped with at least three bores (12) connecting the said surfaces (11, 13), where the said capsule (10) has, optionally, on its outer surface (13) at least two pegs (19), positioned to intersect the plane laying parallel over the entire top of C-shaped cross-section,
- at least three capsule arms (20), where at least one capsule arm (20) has a haptic end (21) for suture-less scleral fixation formed on the one end of the arm duct (22), with the insertion tip (23) formed on the opposite end of the duct (22), where the connecting means (25), which is formed close to the tip (23), is used to connect the said capsule arm (20) and the capsule (10) by inserting the tip (23) into the selected bore (12),
- optionally, one or more filtration blebs (30), or, bleb elements - an outer surface (31) and a base (32), where the base (32) is capable to be connected via its receiving slit (36) with the haptic end (21) of the capsule arm (20), and
- optionally, an artificial iris (90), having slits (91) distributed around the iris to correspond with the pegs (19) geometry and enable iris (90) fixation to the said capsule (10),
- wherein, the arm duct (20) has capillary (24) made through the tip (23) towards the haptic end (21), enabling the effective drainage from the lens capsule (10), situated in the posterior chamber, to the episcleral space directly or through the scleral tunnel in form of a pocket.

2. The intraocular lens capsule kit according to claim 1, wherein the connecting means (25) is formed as a recess on the duct (22), situated close to the insertion tip (23), and where said recess has a diameter that corresponds to the bore (12) diameter, while the duct (22) has a slightly greater diameter than the bore (12) diameter ensuring the locking of the said recess (25) within the capsule (10).

3. The intraocular lens capsule kit according to any of the preceding claims, where the haptic end (21) is elliptic or round and inclined for 15°-45° towards the duct (22), so that the haptic end (21) follows the sclera's curvature and sticks with it.

4. The intraocular lens capsule kit according to claim 2, wherein, the bores (12) are distributed equidistantly over the capsule (10) toroidal body.

5. The intraocular lens capsule kit according to claim 4, wherein the capsule (10) has three bores (12), and three capsule arms (20) with the haptic end.

6. The intraocular lens capsule kit according to any of the claims 1-5, where the said kit comprises a capsule (10) with two pegs (19) and an artificial iris (90) with three slits (91) that match the position of the said pegs (19).

7. The intraocular lens capsule kit according to any of the preceding claims, where the kit further comprises a filtration bleb (30) in the form of the elements - an outer surface (31) equipped with the spacers (33) and a base (32) detachable coupled to the said outer surface (31).

8. The intraocular lens capsule kit according to claim 7, where the base (32) and the surface (31) coupling is achieved via insets (34) formed on the surface edge, which engage the slits (35) formed on the base (32).

9. The intraocular lens capsule kit according to claims 7 or 8, where the bleb (30) is further equipped with one or more holes (37), made on the base (32), for an auxiliary fixation of the said base to the sclera by surgical sutures.

10. The intraocular lens capsule kit according to any of the claims 7-9 where the bleb's interior, formed between the outer surface (31) and the base (32), can be used as a drug reservoir, or depo for slow release of the selected medicament.

11. The intraocular lens capsule kit according to any of the preceding claims, where the kit elements are coated with the suitable agents, selected from anti-inflammatory, anti-glaucomatous, anti-VEGF, or antifibrotic agents or their combinations.

12. Use of the intraocular lens capsule kit featuring drainage according to any of the claims 1-11, for providing the drainage support for the artificial lens situated within the capsule (10) fixed to the sclera via the haptic ends (21) of the capsule arms, and, optionally, equipped with at least one filtration bleb mounted on one of the haptic ends (21) of the capsule arm (20) and with the artificial iris (90), if necessary.
